# EUROPEAN PATENT APPLICATION

(11) **EP 4 311 574 A1**
(43) Date of publication of application: **31.01.2024**
(21) Application number: 23188272.1
(22) Date of filing: 28.07.2023
(51) Int. Cl.: A61P 27/02, C12N 15/113

(54) **PREVENTION AND TREATMENT OF AGE-RELATED MACULAR DEGENERATION THROUGH SUPPRESSION OF CATHEPSIN S EXPRESSION**

(30) Priority: 29.07.2022 KR 20220094997; 21.07.2023 KR 20230095074
(71) Applicant: Chung-Ang University Industry-Academic Cooperation, Seoul 06974 (KR)
(72) Inventor: KIM, Jee Taek, 06001 Seoul (KR)
(74) Representative: Nederlandsch Octrooibureau

(57) **Abstract**

The present invention relates to the prevention and treatment of age-related macular degeneration through the suppression of cathepsin S expression, and retinal pigment epithelial cells in which cathepsin S is knocked down can be usefully used as a target for preventing or treating dry and wet age-related macular degenerations, in that the expression level of inflammatory cytokines and complement activity are reduced even under oxidative stress conditions, and the retinal pigment epithelial cells are not only more effective in suppressing complement activity than NF-κB inhibitors, but also suppress intracellular neovascularization and tube formation.

## Description

### [Technical Field]

The present invention relates to the prevention and treatment of age-related macular degeneration through the suppression of cathepsin S expression.

### [Background]

Age-related macular degeneration (AMD, hereinafter referred to as AMD) is an ophthalmic disease characterized by progressive retinal degeneration of the macula, and corresponds to the most common cause of irreversible central vision loss in the elderly population of developed countries. The global prevalence of AMD is approximately 8.7% among those aged 45 to 85, an early AMD prevalence is 8.0% and a late AMD prevalence corresponds to 0.4%.

Age-related macular degeneration is classified into two forms: dry and wet. Dry age-related macular degeneration refers to a condition that appears in the form of spots (drusen) due to the accumulation of waste products on the retina or a condition in which the accumulation of waste products on the retina leads to atrophy of the retina. Although dry age-related macular degeneration does not induce sudden vision loss, it induces chronic vision loss and may develop into the wet form.

Wet age-related macular degeneration refers to age-related macular degeneration in which abnormal blood vessels (new blood vessels) develop under the macula. These new blood vessels are easily torn and leaked blood or fluid accumulates, resulting in exudate, hemorrhaging, or drusen formation in the macula. Destruction of the macula occurs relatively rapidly, leading to rapid loss of central vision in most cases, and eventually leading to blindness.

Since both forms of age-related macular degeneration cause gradual destruction of the visual cells in the macula, the function of the macula deteriorates and central vision begins to decline over time, and age-related macular degeneration may initially affect only one eye, and the other eye is often affected in many cases. Currently, therapeutic agents for age-related macular degeneration are mainly therapeutic agents for wet age-related macular degeneration, and there are no commercially available therapeutic agents for dry age-related macular degeneration.

Cathepsin S is a cysteine-type protease present in lysosomes, and induces the degradation of damaged proteins through the endosome-lysosomal pathway. In immune cells, it is known that cathepsin S serves to regulate immune responses by degrading invariant chains to activate MHC class II, which is important for antigen presentation, and in cancer cells, it is known to increase the growth of cancer cells by regulating metastasis and angiogenesis, and little is known about the therapeutic use of cathepsin S in relation to age-related macular degeneration despite ongoing research on targets associated with various cancers.

### [Description]

In order to solve the above problems, an object of the present invention is to provide a composition for preventing or treating age-related macular degeneration, an ophthalmic composition, a kit, and a health functional food for preventing or ameliorating age-related macular degeneration as described below.

One object of the present invention is to provide a pharmaceutical composition for preventing or treating age-related macular degeneration, comprising, as an active ingredient, a substance that reduces the expression or activity of cathepsin S (CTSS).

Another object of the present invention is to provide an ophthalmic composition for preventing or treating age-related macular degeneration, which compriss as an active ingredient a substance that reduces the expression or activity of cathepsin S (CTSS).

Still another object of the present invention is to provide a health functional food for preventing or ameliorating age-related macular degeneration, comprising, as an active ingredient, a substance that reduces the expression or activity of cathepsin S.

Another object of the present invention is to provide a kit for preventing or treating age-related macular degeneration, comprising a composition comprising, as an active ingredient, a substance that reduces the expression or activity of cathepsin S, and instructions.

However, the technical problems which the present invention intends to solve are not limited to the technical problems that have been mentioned above, and other technical problems, which have not been mentioned, will be clearly understood by a person with ordinary skill in the art to which the present invention pertains from the following description.

To achieve the above objects, the present invention provides a pharmaceutical composition for preventing or treating age-related macular degeneration, comprising, as an active ingredient, a substance that reduces the expression or activity of cathepsin S (CTSS).

According to an exemplary embodiment of the present invention, the substance may be any one or more selected from the group consisting of small interfering RNA (siRNA), short hairpin RNA (shRNA), micro RNA (miRNA), antisense oligonucleotides (ASOs), Clustered Regularly Interspaced Short Palindromic Repeats (CRISPR/Cas), natural products, proteins, peptidomimetics, antibodies, exosomes, and compounds, but is not limited thereto.

According to one embodiment of the present invention, the age-related macular degeneration may be age-related macular degeneration under oxidative stress conditions, but is not limited thereto.

According to one embodiment of the present invention, the age-related macular degeneration may be dry age-related macular degeneration or wet age-related macular degeneration, but is not limited thereto.

According to one embodiment of the present invention, the composition may be characterized by any one or more of the following, but is not limited thereto:
inhibition of inflammatory cytokines and complement activity in the retina;
inhibition of intravascular endothelial production; and
inhibition of angiogenesis.

According to an exemplary embodiment of the present invention, the inflammatory cytokine may be any one or more selected from the group consisting of TNF-α, IL-1β, and MCP-1, but is not limited thereto.

According to an exemplary embodiment of the present invention, the complement may be any one or more selected from the group consisting of C3, C5, C3a, C5a, and C5b-9, but is not limited thereto.

The present invention provides an ophthalmic composition for preventing or treating age-related macular degeneration comprising a substance that reduces the expression or activity of cathepsin S as an active ingredient.

According to one embodiment of the present invention, the composition may be an eye drop composition, but is not limited thereto.

The present invention provides a health functional food for preventing or ameliorating age-related macular degeneration, comprising, as an active ingredient, a substance that reduces the expression or activity of cathepsin S.

According to an exemplary embodiment of the present invention, the substance may be any one or more selected from the group consisting of small interfering RNA (siRNA), short hairpin RNA (shRNA), micro RNA (miRNA), antisense oligonucleotides (ASOs), Clustered Regularly Interspaced Short Palindromic Repeats (CRISPR/Cas), natural products, proteins, peptidomimetics, antibodies, exosomes, and compounds, but is not limited thereto.

The present invention provides a kit for preventing or treating age-related macular degeneration, comprising a composition comprising, as an active ingredient, a substance that reduces the expression or activity of cathepsin S, and instructions.

Furthermore, the present invention provides a method for preventing, alleviating, or treating age-related macular degeneration, the method comprising administering a composition comprising, as an active ingredient, the substance that reduces the expression or activity of cathepsin S.

Further, the present invention provides a use of a composition comprising, as an active ingredient, the substance that reduces the expression or activity of cathepsin S, for preventing, alleviating or treating age-related macular degeneration.

In addition, the present invention provides a use of a composition comprising, as an active ingredient, the substance that reduces the expression or activity of cathepsin S, for preparing a preventive, alleviating, or therapeutic agent for age-related macular degeneration.

In addition, the present invention provides a use of a composition comprising, as an active ingredient, the substance that reduces the expression or activity of cathepsin S, for preparing an ophthalmic preparation for preventing or treating age-related macular degeneration.

According to prevention and treatment of age-related macular degeneration through suppression of cathepsin S expression, retinal pigment epithelial cells in which cathepsin S is knocked down have reduced expression levels of inflammatory cytokines and complement activity even under oxidative stress conditions, and complement compared to NF-κB inhibitors. It can be usefully used as a substance for preventing or treating dry and wet age-related macular degeneration in that it not only has an excellent activity inhibitory effect, but also inhibits intracellular neovascularization and tube formation.

The present inventors completed the present invention by confirming the effect of preventing or treating age-related macular degeneration by cathepsin S knockdown cells and cathepsin S siRNA treatment in one embodiment according to the present invention. Therefore, the present invention provides a pharmaceutical composition for preventing or treating age-related macular degeneration, comprising, as an active ingredient, a substance that reduces the expression or activity of cathepsin S (CTSS).

In the present invention, "reduction of expression" means any action that reduces the expression of a gene, specifically gene knock-out (knock-down), RNA interference;RNAi, gene DNA sequence deletion, duplication, inversion, or it may be achieved by introducing a mutation such as replacement, but is not limited thereto.

In the present invention, "reduced activity" means that one or more functions generated by cathepsin S are inhibited, and the functions cause age-related macular degeneration, progress or worsen symptoms of age-related macular degeneration, or progress rate of age-related macular degeneration. It may be a function related to the occurrence/deterioration of symptoms of age-related macular degeneration, such as increasing, but is not limited thereto.

In an exemplary embodiment of the present invention, the substance may be any one or more selected from the group consisting of small interfering RNA (siRNA), short hairpin RNA (shRNA), micro RNA (miRNA), antisense oligonucleotides (ASOs), Clustered Regularly Interspaced Short Palindromic Repeats (CRISPR/Cas), natural products, proteins, peptidomimetics, antibodies, exosomes, and compounds, but is not limited thereto.

As used herein, "polynucleotide" or "nucleic acid" refers to deoxyribonucleic acid (DNA) or ribonucleic acid (RNA) in either a single- or double-stranded form. Unless otherwise limited, the term also encompasses known analogs of natural nucleotides which are hybridized to a nucleic acid in a manner similar to naturally occurring nucleotides.

As used herein, "siRNA" refers to RNA molecules, generally 17 to 24 bp in length, which can interfere with and inhibit the expression of a gene comprising a nucleic acid sequence complementary to the sequence of siRNA in a process called RNA interference, but is not limited thereto. The siRNA comprises both single-stranded siRNA and double-stranded siRNA, but is not limited thereto. Double-stranded siRNA is derived from longer double-stranded RNA molecules (dsRNA), and dsRNA may be cleaved by an endoribonuclease (Dicer) to form double-stranded siRNA, and single-stranded siRNA may be formed in a nucleoprotein complex (RISC), but is not limited thereto.
comprisIn the present invention, methods commonly used in the art can be applied to the synthesis or separation and purification method of siRNA, the method for delivering siRNA, etc., and, for example, various plasmids that can be expressed in eukaryotic cells, non-viral vectors such as liposomes, etc, but is not limited thereto.

In the present invention, "shRNA" is a short hairpin RNA having a loop structure that induces the NAi phenomenon and refers to a substance that induces mRNA degradation by complementarily binding to target mRNA. The shRNA may be microRNA-adapted shRNA or simple hairpin shRNA, but is not limited thereto, and comprises all forms modified to improve the function of shRNA.

In the present invention, "miRNA" is generally a single-stranded RNA molecule with a length of about 22 nucleotides (nt), and refers to a microregulator that binds to the 3'UTR of target mRNA to suppress the expression of the corresponding gene by forming a complex with an Argonaute protein (AGO).

In the present invention, "antisense oligonucleotide" is a drug with the potential to realize personalized medicine together with an RNAi therapeutic agent and a single-stranded nucleic acid polymer synthesized with 12 to 25 nucleotides on average, and may regulate protein expression through various mechanisms with specific sequence complementarity to a target RNA. For the antisense oligonucleotide, single-stranded ASOs may regulate gene expression chemically or by binding sites and targets, or affect mRNA splicing through various mechanisms. Complementary binding of ASO to a pre-mRNA target site may activate ribonuclease H (RNAase H), which hydrolyzes the RNA strands of an RNA-DNA heteroduplex to suppress target mRNA degradation and protein expression, but is not limited thereto, and the ASO may induce steric hindrance upon binding to mRNA to interfere with the interaction with RNA-binding proteins.

In the present invention, the antisense oligonucleotide may be used by being modified with phosphorothioate (PS), a phosphorodiamidate morpolino oligonucleotide (PMO), a peptide nucleic acid (PNA), a locked nucleic acid (LNA), ribose modifications, and the like, but is not limited thereto, and comprises all modifications to which techniques generally applicable in the art are applied to improve functionality.

In the present invention, "CRISPR/Cas" may mean 'guide RNA (gRNA)' made from RNA and 'Cas,' for example, it may be Cas9, Cas11, or Cas12, but is not limited thereto, and Cas may comprise a broad meaning comprising all types of enzymes that can be used in the CRIPR system.As used herein, "Cas9" refers to "CRISPR-Cas9," and CRISPR-Cas9 recognizes, cuts and edits a specific nucleotide sequence to be used as a third-generation gene scissors, and may be usefully used to insert a specific gene at a target site in the genome or to simply, quickly and efficiently perform a manipulation to stop the activity of a specific gene.

Cas9 protein or gene information may be obtained from a publicly-known database such as the GenBank of the National Center for Biotechnology Information (NCBI), but the source is not limited thereto. Furthermore, the Cas9 protein may be appropriately linked with additional domains by those skilled in the art according to its purpose.

In the present invention, the Cas9 protein comprises not only wild-type Cas9, but also all mutants of Cas9 as long as they have the function of a nuclease for gene editing, and the origin of the Cas9 protein is not limited, and as a non-limiting example thereof, the Cas9 protein may be derived from Streptococcus pyogenes, Francisella novicida, Streptococcus thermophilus, Legionella pneumophila, Listeria innocua, or Streptococcus mutans, and may be appropriately selected and used by those skilled in the art, but is not limited thereto.

In the present invention, "gRNA" is a single-stranded RNA that locates the position of a specific DNA to be edited and serves to guide a Cas protein to the target DNA, and the guide RNA may comprise a sequence adjacent to a protospacer adjacent motif (PAM) locus and complementary to a 10 to 25 bp base sequence of the DNA to be edited. The guide RNA may have 1 to 3 additional nucleotides, more specifically 2 or 3 nucleotides, in front of the 5' site of a sequence capable of base-pairing with the complementary strand of a target DNA sequence. In addition, the guide RNA may comprise a portion having a sequence complementary to a sequence in the target DNA (also referred to as a spacer region, a target DNA recognition sequence, a base pairing region, and the like) and a hairpin structure for Cas protein binding. More specifically, the guide RNA may comprise a portion having a sequence complementary to a sequence in the target DNA, a hairpin structure for Cas protein binding, and a terminator sequence, but is not limited thereto.

The gRNA of the present invention may target a cathepsin S gene and may be configured in pairs to simultaneously target different regions of the gene, but is not limited thereto.

In the present invention, "natural product" refers to a product derived from organisms such as animals and plants that live on land and in the sea, and a product derived from organisms such as cells or tissue culture products of organisms, and may mean a natural product as it is without the addition of artificial means. A natural extract is not particularly limited as long as it is an extract from natural products, and may be, for example, a plant extract, an animal extract, a microbial extract, a mineral extract, and the like.

Further, the natural product may mean itself, but is not limited thereto, and may mean all modified forms of the natural product comprising pharmacologically active ingredients. For example, the natural product may be an extract or fractions thereof, a fermented product, a juice, and the like, but is not limited thereto.

Conditions such as a solvent for the extract are not particularly limited, and all conditions commonly used by those skilled in the art may be applied according to the characteristics of the natural product or so as to comprise pharmacologically active ingredients.

The method for fractionating the natural product into fractions is not particularly limited as long as it is a typically used method for fractionating an extract, and as a non-limiting example thereof, the extract may be fractionated using any one or more methods selected from the group consisting of a chromatographic separation method, a powder-liquid separation method using solubility differences, a separation method using specific gravity differences, a centrifugal separation method using density differences, a solid sample extraction method, and a separation method using color differences.

As used herein, "protein" is interchangeably used with "polypeptide" or "peptide," and refers to, for example, a polymer of amino acid residues as generally found in proteins in a natural state, and may be produced by a recombinant expression vector, but is not limited thereto.

As used herein, "recombinant expression vector" refers to a bacterial plasmid, a phage, a yeast plasmid, a plant cell virus, a mammalian cell virus or other vectors. In general, any plasmid or vector may be used as long as it can be replicated and stabilized in a host. The recombinant expression vector of the present may preferably comprise a promoter which is a transcription initiation factor to which RNA polymerase binds, any sequence for regulating transcription, a sequence encoding a suitable mRNA ribosome binding site and a sequence for regulating the termination of transcription and translation, a terminator, and the like, and the recombinant expression vector and an expression vector comprising appropriate transcriptional/translational control signals may be constructed by methods well known to those skilled in the art. comprisIn addition, a tag gene for increasing the yield of a recombinant protein, a tag gene for maintaining the structural stability of a recombinant protein, a tag gene for easily isolating a recombinant protein, a selection marker gene such as an antibiotic resistance gene for selecting a transformant, and the like may be further comprised, the tag for easy isolation may comprise, but is not limited to, an Avi tag, a Calmodulin tag, a polyglutamate tag, an E tag, a FLAG tag, a HA tag, a His tag, a Myc tag, an S tag, a SBP tag, an IgG-Fc tag, a CTB tag, a Softag 1 tag, a Softag 3 tag, a Strep tag, a TC tag, a V5 tag, a VSV tag, an Xpress tag, and the like, representative examples of the selection marker gene comprise herbicide resistance genes such as glyphosate or phosphinothricin, antibiotic resistance genes such as kanamycin, G418, Bleomycin, hygromycin, and chloramphenicol, aadA genes, and the like, representative examples of the promoter comprise a pEMU promoter, a MAS promoter, a histone promoter, a Clp promoter, a cauliflower-mosaic-virus-derived 35S promoter, a cauliflower-mosaic-virus-derived 19S RNA promoter, an actin protein promoter of plant, a ubiquitin protein promoter, a cytomegalovirus (CMV) promoter, a simian virus 40 (SV40) promoter, a respiratory syncytial virus (RSV) promoter, an elongation factor-1 alpha (EF-1a) promoter, and the like, representative examples of the terminator comprise a nopaline synthase (NOS), a rice amylase RAmy1 A terminator, a phaseolin terminator, a terminator of an octopine gene of Agrobacterium tumefaciens, a rrnB 1B2 terminator of *E. coli,* and the like, but the type of gene to be added is not limited as long as it is a type that has been used in the production of a recombinant protein in the related art.

Any method known in the art may be used to transform into a recombinant vector or to produce a target protein from a transformed transformer into a recombinant vector, but the transformer may be an animal or a plant depending on the characteristics of the recombinant vector.

As used herein, a "peptidomimetic" refers to one designed to mimic a biologically active peptide, and may be a non-natural amino acid or another unique compound to stabilize a structure or alter biological activity, but is not limited thereto. The peptidomimetic may be modified to enhance the target specificity and stability of the peptide and decrease the degradability, and the like of the peptide. For example, bending may be limited such that the conformational mobility of the peptide may be limited, or a non-natural compound may be comprised in the structure to make it less likely to be degraded by enzymes, but the modification is not limited thereto.

In the present invention, "antibody" is a term interchangeable with "antibody fragment" and means an antibody that is naturally present in the body, but is not limited thereto, and comprises all antibodies obtained from human antibody phage libraries and human antibody-producing transgenic animals prepared by the advancement of genetic engineering, cell engineering, and developmental engineering techniques, and the like. In addition, the antibody fragment comprises a part of an antibody, and refers to one that may perform the same function as an antibody, and may be a peptide such as, for example, Fab, Fab', F(ab')2, scFv, a diabody, dsFv and CDR, and the like, but is not limited thereto. The antibody fragment may be prepared by applying a method commonly performed in the art.

As used herein, "exosomes" refer to those excreted from somatic cells derived from human tissue, comprising neurospheres, fibroblasts, epithelial cells, muscle cells, heart cells, kidney cells, nerve cells, hair cells, hair root cells, follicle cells, epithelial cells, beta cells, gastric mucosa cells, goblet cells, G cells, immune cells, pericytes, mast cells, endothelial cells, but are not limited thereto. In addition, the exosomes may be excreted from cells extracted from solutions excreted from the human body such as urine, saliva, sweat, blood, and the like, but are not limited thereto, and may mean those excreted from bone marrow-derived stem cells such as neural cord blood, adipose-derived stem cells, adult stem cells, embryonic stem cells, pluripotent stem cells such as iPSCs, placental stem cells, and the like, but are not limited thereto. In order to improve the secretion or functionality of exosomes, such as facilitating exosome excretion or increasing the number of exosomes excreted as described above, physical stimuli such as electrical stimulation, heat, sound waves, lasers, oxidative stress, LED light, chemical stimuli through chemical substance treatments, biological stimuli through treatments with substances such as enzymes acting on *in vivo* mechanisms, and the like can be applied, but are not limited thereto. In the present invention, the size of exosomes may be the size of general exosomes, but is not limited thereto, may vary depending on the excretion method, stimulation method, or culture method, and may vary depending on the amount, type and the like of substance to be loaded.

In the present invention, any method of stimulating, secreting and obtaining exosomes can be applied as long as it is natural for those skilled in the art, and is not limited thereto.

In the present invention, a "compound" is a substance made by chemically bonding two or more different elements, and comprises both organic compounds and inorganic compounds. The compound of the present invention may mean any chemical substance capable of suppressing the expression or activity of cathepsin S.

In the present invention, a substance that reduces the expression or activity of cathepsin S may be delivered to target cells by a method or vehicle typically used in the art. For example, the material may be delivered by a vector, comprisbut are not limited thereto. In addition, the substance of the present invention may be delivered by a generally applicable method or vehicle according to the dose and cycle of the substance, the age, sex, and clinical symptoms of a user, the disease progression, and the like, but is not limited thereto.

As used herein, the "age-related macular degeneration" refers to a degenerative disease that occurs in the macula of the eye tissue and induces vision deterioration.

The age-related macular degeneration may be classified into dry and wet types, and there is no particular treatment method for dry age-related macular degeneration (nonexudative or atrophic), but examples of a method for treating wet age-related macular degeneration (neovascular and exudative) comprise intraocular injection, photodynamic therapy, laser photocoagulation, and the like and taking antioxidants or wearing sunglasses may be recommended to reduce the risk of age-related macular degeneration progression, but the treatment method is not limited thereto.

Both types of age-related macular degeneration (AMD) begin with the dry type and develop to wet AMD in some patients (about 10%), and about 85% of AMD patients have only the dry type. Although relatively few patients exhibit the wet type, 80 to 90% of severe vision loss due to AMD may be caused by wet AMD.

In the case of dry AMD, macular tissue becomes thin and cells disappear, waste products accumulated in rod cells and cone cells may produce deposits called drusen (yellow spots) on the retina (the transparent structure behind the eyeball that is sensitive to light), and dry AMD may affect both eyes at the same time, and there may be no signs of scarring or hemorrhaging or other fluid leaks in the macula.

Wet AMD occurs when abnormal blood vessels grow in the choroid (the layer of blood vessels between the retina and the outer white layer of the eye called the sclera) under the macula and leak blood and fluid (hence described as "wet"), a mass of scar tissue may occur under the macula, and although wet AMD first occurs in one eye, both eyes may eventually be affected.

In this invention, age-related macular degeneration comprises all age-related macular degeneration caused by internal or external factors, and in the embodiment of this invention, the age-related macular degeneration may be age-related macular degeneration under oxidative stress conditions, but is not limited to it, and also in the embodiment of this invention, the age-related macular degeneration may be dry age-related macular degeneration or wet age-related macular degeneration, but is not limited thereto.

In an embodiment of the present invention, the composition may be characterized by at least one of the following, but is not limited thereto:
Inhibit inflammatory cytokines and complement activity in the retina;
inhibit endovascular membrane production; and
inhibition of neovascularization.

In an exemplary embodiment of the present invention, the inflammatory cytokine may be any one or more selected from the group consisting of TNF-α, IL-1β, and MCP-1, but is not limited thereto.

In an exemplary embodiment of the present invention, the complement may be any one or more selected from the group consisting of C3, C5, C3a, C5a, and C5b-9, but is not limited thereto.

The composition for preventing or treating age-related macular degeneration according to the present invention may be used in combination with an existing age-related macular degeneration therapeutic agent or treatment method, but is not limited thereto.

The pharmaceutical composition for prevention or treatment according to the present invention may further comprise an appropriate carrier, excipient, and diluent, which are typically used to prepare a pharmaceutical composition. The excipient may be, for example, one or more selected from the group consisting of a diluent, a binder, a disintegrant, a lubricant, an adsorbent, a moisturizer, a film-coating material, and a controlled release additive.

The pharmaceutical composition according to the present invention may be used by being formulated into the form of a powder, a granule, a sustained-release granule, an enteric granule, a liquid, a collyrium, an elixir, an emulsion, a suspension, a spirit, a troche, aromatic water, a limonade, a tablet, a sustained-release tablet, an enteric tablet, a sublingual tablet, a hard capsule, a soft capsule, a sustained-release capsule, an enteric capsule, a pill, a tincture, a soft extract agent, a dry extract agent, a fluid extract agent, an injection, a capsule, a perfusate, an external preparation such as a plaster, a lotion, a paste, a spray, an inhalant, a patch, a sterilized injection solution, or an aerosol, and the external preparation may have a formulation such as a cream, a gel, a patch, a spray, an ointment, a plaster, a lotion, a liniment, a paste or a cataplasma.

Examples of a carrier, an excipient or a diluent which may be comprised in the composition according to the present invention comprise lactose, dextrose, sucrose, an oligosaccharide, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methyl hydroxybenzoate, propyl hydroxybenzoate, talc, magnesium stearate, and mineral oil.

When the pharmaceutical composition is prepared, the pharmaceutical composition is prepared using a diluent or excipient, such as a filler, an extender, a binder, a wetting agent, a disintegrant, and a surfactant, which are commonly used.

As an additive of the tablet, powder, granule, capsule, pill, and troche according to the present invention, it is possible to use an excipient such as corn starch, potato starch, wheat starch, lactose, sucrose, glucose, fructose, D-mannitol, precipitated calcium carbonate, synthetic aluminum silicate, calcium monohydrogen phosphate, calcium sulfate, sodium chloride, sodium hydrogen carbonate, purified lanolin, microcrystalline cellulose, dextrin, sodium alginate, methyl cellulose, carboxymethyl cellulose sodium, kaolin, urea, colloidal silica gel, hydroxypropyl starch, hydroxypropyl methylcellulose (HPMC), HPMC 1928, HPMC 2208, HPMC 2906, HPMC 2910, propylene glycol, casein, calcium lactate, and Primojel; and a binder such as gelatin, arabic gum, ethanol, agar powder, cellulose acetate phthalate, carboxymethyl cellulose, carboxymethyl cellulose calcium, glucose, purified water, sodium caseinate, glycerin, stearic acid, carboxymethyl cellulose sodium, methylcellulose sodium, methylcellulose, microcrystalline cellulose, dextrin, hydroxycellulose, hydroxypropyl starch, hydroxymethyl cellulose, purified shellac, starch, hydroxypropyl cellulose, hydroxypropyl methyl cellulose, polyvinyl alcohol, and polyvinylpyrrolidone, and it is possible to use a disintegrant such as hydroxypropyl methyl cellulose, corn starch, agar powder, methyl cellulose, bentonite, hydroxypropyl starch, carboxymethyl cellulose sodium, sodium alginate, carboxymethyl cellulose calcium, calcium citrate, sodium lauryl sulfate, silicic anhydride, 1-hydroxypropyl cellulose, dextran, an ion exchange resin, polyvinyl acetate, formaldehyde-treated casein and gelatin, alginic acid, amylose, guar gum, sodium bicarbonate, polyvinylpyrrolidone, calcium phosphate, gelled starch, arabic gum, amylopectin, pectin, sodium polyphosphate, ethyl cellulose, sucrose, magnesium aluminum silicate, a D-sorbitol solution, and light anhydrous silicic acid; and a lubricant such as calcium stearate, magnesium stearate, stearic acid, hydrogenated vegetable oil, talc, lycopodium powder, kaolin, Vaseline, sodium stearate, cacao butter, sodium salicylate, magnesium salicylate, polyethylene glycol (PEG) 4000, PEG 6000, liquid paraffin, hydrogenated soybean oil (Lubriwax), aluminum stearate, zinc stearate, sodium lauryl sulfate, magnesium oxide, Macrogol, synthetic aluminum silicate, silicic anhydride, higher fatty acids, higher alcohols, silicone oil, paraffin oil, polyethylene glycol fatty acid ether, starch, sodium chloride, sodium acetate, sodium oleate, dl-leucine, and light anhydrous silicic acid.

As an additive for liquid formulation according to the present invention, it is possible to use water, diluted hydrochloric acid, diluted sulfuric acid, sodium citrate, sucrose monostearate, polyoxyethylene sorbitol fatty acid esters (Tween esters), polyoxyethylene monoalkyl ethers, lanolin ether, lanolin esters, acetic acid, hydrochloric acid, aqueous ammonia, ammonium carbonate, potassium hydroxide, sodium hydroxide, prolamin, polyvinyl pyrrolidone, ethyl cellulose, carboxymethyl cellulose sodium, and the like.

In a syrup according to the present invention, a solution of sucrose, other sugars or sweeteners, and the like may be used, and a fragrance, a colorant, a preservative, a stabilizer, a suspending agent, an emulsifier, a thickener, and the like may be used if necessary.

Purified water may be used for the emulsion according to the present invention, and an emulsifier, a preservative, a stabilizer, a fragrance, and the like may be used if necessary.

In the suspension according to the present invention, a suspending agent such as acacia, tragacanth, methyl cellulose, carboxymethyl cellulose, carboxymethyl cellulose sodium, microcrystalline cellulose, sodium alginate, hydroxypropyl methyl cellulose (HPMC), HPMC 1828, HPMC 2906, and HPMC 2910 may be used, and a surfactant, a preservative, a colorant, and a fragrance may be used, if necessary.

The injection according to the present invention may comprise: a solvent such as distilled water for injection, a 0.9% sodium chloride injection, a Ringer's injection, a dextrose injection, a dextrose+sodium chloride injection, PEG, a lactated Ringer's injection, ethanol, propylene glycol, non-volatile oil-sesame oil, cottonseed oil, peanut oil, corn oil, ethyl oleate, isopropyl myristate, or benzoic acid benzene; a solubilizing aid such as sodium benzoate, sodium salicylate, sodium acetate, urea, urethane, monoethyl acetamide, butazolidin, propylene glycol, Tweens, nijungtinateamide, hexamine, or dimethylacetamide; a buffer such as a weak acid or salt thereof (acetic acid or sodium acetate), a weak base or salt thereof (ammonia or ammonium acetate), an organic compound, a protein, albumin, peptone, or a gum; an isotonic agent such as sodium chloride; a stabilizer such as sodium bisulfite (NaHSO₃), carbon dioxide gas, sodium metabisulfite (Na₂S₂O₅), sodium sulfite (Na₂SO₃), nitrogen gas (N₂), or ethylenediaminetetraacetic acid; a sulfating agent such as 0.1% sodium bisulfide, sodium formaldehydesulfoxylate, thiourea, disodium ethylenediaminetetraacetate, or acetone sodium bisulfite; an analgesic such as benzyl alcohol, chlorobutanol, procaine hydrochloride, glucose, or calcium gluconate; and a suspending agent such as carboxymethyl cellulose sodium, sodium alginate, Tween 80, or aluminum monostearate.

In a suppository according to the present invention, it is possible to use a base such as cacao butter, lanolin, Witepsol, polyethylene glycol, glycerogelatin, methylcellulose, carboxymethyl cellulose, a mixture of stearic acid and oleic acid, Subanal, cottonseed oil, peanut oil, palm oil, cacao butter+cholesterol, lecithin, ranetwax, glycerol monostearate, Tween or Span, Imhausen, monolen (propylene glycol monostearate), glycerin, Adeps solidus, Buytyrum Tego-G, Cebes Pharma 16, hexalide base 95, Cotomar, Hydroxote SP, S-70-XXA, S-70-XX75(S-70-XX95), Hydrokote 25, Hydrokote 711, idropostal, Massa estrarium (A, AS, B, C, D, E, I, T), Massa-MF, Masupol, Masupol-15, Neosupostal-ene, Paramound-B, Suposhiro (OSI, OSIX, A, B, C, D, H, L), suppository base IV types (AB, B, A, BC, BBG, E, BGF, C, D, 299), Supostal (N, Es), Wecobee (W, R, S, M ,Fs), or a tegester triglyceride base (TG-95, MA, 57).

A solid formulation for oral administration comprises a tablet, a pill, a powder, a granule, a capsule, or the like, and the solid formulation is prepared by mixing at least one excipient, for example, starch, calcium carbonate, sucrose or lactose, gelatin, and the like, with an extract. Further, in addition to a simple excipient, lubricants such as magnesium stearate and talc are also used.

A liquid formulation for oral administration corresponds to a suspension, a liquid for internal use, an emulsion, a syrup, or the like, and the liquid formulation may comprise, in addition to water and liquid paraffin which are simple commonly used diluents, various excipients, for example, a wetting agent, a sweetener, a fragrance, a preservative, and the like. Examples of a formulation for parenteral administration comprise an aqueous sterile solution, a non-aqueous solvent, a suspension, an emulsion, a freeze-dried preparation, and a suppository. As the non-aqueous solvent and the suspension, it is possible to use propylene glycol, polyethylene glycol, a vegetable oil such as olive oil, an injectable ester such as ethyl oleate, and the like.

The pharmaceutical composition according to the present invention is administered in a pharmaceutically effective amount. In the present invention, "pharmaceutically effective amount" means an amount sufficient to treat diseases at a reasonable benefit/risk ratio applicable to medical treatment, and an effective dosage level may be determined according to factors comprising the type of disease of a patient, the severity of the disease, the activity of drugs, sensitivity to drugs, administration time, administration route, excretion rate, treatment period, and simultaneously used drugs, and other factors well known in the medical field.

The pharmaceutical composition according to the present invention may be administered as an individual therapeutic agent or in combination with other therapeutic agents, may be administered sequentially or simultaneously with therapeutic agents in the related art, and may be administered in a single dose or multiple doses. It is important to administer the composition in a minimum amount that can obtain the maximum effect without any side effects, in consideration of all the aforementioned factors, and this amount may be easily determined by a person with ordinary skill in the art to which the present invention pertains.

The pharmaceutical composition of the present invention may be administered to a subject in need via various routes. All methods of administration may be expected, but the pharmaceutical composition may be administered by, for example, oral administration, subcutaneous injection, peritoneal administration, intravenous injection, intramuscular injection, paraspinal space (intradural) injection, sublingual administration, buccal administration, intrarectal insertion, intravaginal injection, ocular administration, ear administration, nasal administration, inhalation, spraying via the mouth or nose, skin administration, transdermal administration, and the like.

The pharmaceutical composition of the present invention is determined by the type of drug that is an active ingredient, as well as various related factors such as the disease to be treated, the route of administration, the age, sex, and body weight of a patient, and the severity of the disease.

As used herein, the "subject" refers to a subject in need of treatment of a disease, and more specifically, refers to a mammal such as a human or a non-human primate, a mouse, a rat, a dog, a cat, a horse, and a cow.

The "administration" as used herein refers to the provision of a predetermined composition of the present invention to a subject in need thereof by any suitable method.

As used herein, the "prevention" refers to all actions that suppress or delay the onset of a target disease, and the "treatment" refers to all actions that ameliorate or beneficially change a target disease and the resulting metabolic abnormalities by administration of the pharmaceutical composition according to the present invention, and the "amelioration" refers to all actions that reduce a target disease and associated parameters, for example, the severity of symptoms, by administration of the composition according to the present invention.

The present invention provides an ophthalmic composition for preventing or treating age-related macular degeneration comprising a substance that reduces the expression or activity of cathepsin S as an active ingredient.

According to one embodiment of the present invention, the composition may be an eye drop composition, but is not limited thereto.

The ophthalmic composition of the present invention is used to prevent or treat age-related macular degeneration, and the ophthalmic composition for prevention or treatment in the present invention may be used in the same manner as the ophthalmic pharmaceutical composition. In addition, ophthalmic compositions may be used to treat and/or prevent microbial infections. Microorganisms can be bacteria, viruses, fungi, or amoeba, parasites, or a combination thereof. Bacteria may be, but are not limited to, mycobacteria. In addition, it can be used to prevent or treat diseases such as conjunctivitis, corneal abrasions, inflammatory ulcer keratitis, epithelial keratitis, substrate keratitis, and herpesvirus-related keratitis, in addition to the use of the above treatment.

It may compris additives commonly comprised in ophthalmic preparations, for example, additives such as buffers, appearances, and pH adjusters.

Sodium hydrogen phosphate (or a hydrate thereof), sodium dihydrogen phosphate (or a hydrate thereof), and the like may be used as the buffer, but are not limited thereto. The amount of the buffer used may be appropriately selected by those skilled in the art, and for example, sodium hydrogen phosphate (or a hydrate thereof) and sodium hydrogen phosphate (or a hydrate thereof) may be used in the range of 0.80 w/v% to 3.00 w/v%, respectively.

Sodium chloride, potassium chloride, boric acid, borax, and the like may be used as the initiator, but this invention is not limited thereto. The amount of use of the appearing agent may be appropriately selected by those skilled in the art. For example, when sodium chloride is used, it can be used in the range of 0.75 w/v% to 0.95 w/v%. When sodium chloride, boric acid, and borax are used, they may be used in the range of 0.05 w/v% to 1.00 w/v%, respectively.

The pH control agent may be added to adjust the pH of the obtained ophthalmic solution composition to an appropriate range, for example, pH 5.0 to 8.0, preferably about pH 7.0, and generally hydrochloric acid or sodium hydroxide may be used. The pH regulator may be used in an amount required to obtain an appropriate range of pH, but is not limited thereto.

The ophthalmic composition of the present invention may be prepared by being comprised in general ophthalmic preparations at an optimal concentration that can most increase the effect and does not cause side effects. At this time, the ophthalmic preparation may be mixed in an aqueous medium, and the aqueous medium may be sterilized purified water, injection water, or the like suitable for the preparation of the ophthalmic preparation, but is not limited thereto.

In addition, the ophthalmic composition of this invention may be in the form of a solution, suspension, emulsion, ointment, cream, gel, or release-controlled/western medium, for example, a contact lens solution, eye drops, tears, etc., and preferably, the composition may be an ophthalmic composition, but is not limited thereto.

In addition, the ophthalmic composition of this invention can determine the treatment cycle, amount, concentration, and form of the drug, along with various related factors such as the disease to be treated, the patient's age, gender, weight, and the severity of the disease.

The present invention provides a health functional food for preventing or ameliorating age-related macular degeneration, comprising, as an active ingredient, a substance that reduces the expression or activity of cathepsin S.

In an exemplary embodiment of the present invention, the substance may be any one or more selected from the group consisting of small interfering RNA (siRNA), short hairpin RNA (shRNA), micro RNA (miRNA), antisense oligonucleotides (ASOs), Clustered Regularly Interspaced Short Palindromic Repeats (CRISPR/Cas), natural products, proteins, peptidomimetics, antibodies, exosomes, and compounds, but is not limited thereto.

The health food and health functional food composition comprising the active substance according to the present invention may be added to food as it is or may be used together with other foods or food ingredients, and may be appropriately used according to a general method. Further, the mixing amount of the active substance may be suitably determined depending on its purpose of use (for example, for prevention or amelioration).

In general, the composition in health foods and health functional foods may be added in an amount of 0.1 to 90 parts by weight of the total food weight. However, in the case of long-term intake for the purpose of maintaining health or for the purpose of health control, the amount may be under than the above range, and the active ingredient according to the present invention may be used in an amount above than the above range in terms of safety.

Other ingredients are not particularly limited, other than that the health food and health functional food composition of the present invention compriss the active substance of the present invention as an essential ingredient at an indicated ratio, and various flavoring agents like those of a typical beverage, natural carbohydrates, and the like may be comprised as additional ingredients. Examples of the natural carbohydrates comprise typical sugars such as monosaccharides, for example, glucose, fructose and the like; disaccharides, for example, maltose, sucrose and the like; and polysaccharides, for example, dextrin, cyclodextrin and the like, and sugar alcohols such as xylitol, sorbitol, and erythritol. As flavoring agents in addition to those described above, a natural flavoring agent (thaumatin), a stevia extract (for example, rebaudioside A, glycyrrhizin and the like), and a synthetic flavoring agent (saccharin, aspartame and the like) may be used. The proportion of the natural carbohydrate may be generally about 1 to 20 parts by weight, and preferably about 5 to 12 g per 100 g of the health functional food composition of the present invention, but is not limited thereto.

The health food and health functional food composition comprising the active substance of the present invention may comprise various nutrients, vitamins, minerals (electrolytes), flavoring agents such as synthetic flavoring agents and natural flavoring agents, colorants and thickening agents (cheese, chocolate, and the like), pectic acid and salts thereof, alginic acid and salts thereof, organic acids, protective colloid thickeners, pH adjusting agents, stabilizers, preservatives, glycerin, alcohols, carbonating agents used in carbonated beverages, or the like, in addition to the additives. In addition, the health food and health functional food composition of the present invention may comprise flesh for preparing natural fruit juice, fruit juice drinks, and vegetable drinks.

These ingredients may be used independently or in combination, and are generally selected within a range of 0.1 to about 20 parts by weight per 100 parts by weight of the health food and health functional food composition comprising the active substance of the present invention, but the range is not limited thereto.

This invention provides a kit for preventing or treating age-related macular degeneration, comprising a composition comprising, as an active ingredient, a substance that reduces the expression or activity of cathepsin S, and instructions.

In addition to the above configuration, the "kit" of the present invention may comprise other components, devices, materials, etc. commonly required for storing, managing, and improving the effect of the composition of the present invention. In addition, all configurations comprised in the kit can be used without limitation more than once, and there is no limit after using each material, and the application of each material can proceed simultaneously or microscopically.

The kit of the present invention may comprise a compriser in addition to the preparation and the description. The compriser may serve to package the configuration, and may serve to store and fix the configuration. The material of the compriser may take the form of, for example, a bottle, a tub, a small bag, an envelope, a tube, an ampoule, or the like, and may be partially or entirely formed of plastic, glass, paper, foil, wax, etc. The compriser may be equipped with a stopper that is initially part of the compriser or can be attached to the compriser by mechanical, adhesive, or other means, and may also be equipped with a stopper that can access the contents by an injection needle. The kit may comprise an external package, and the external package may comprise instructions on the use of components, but is not limited thereto.

With respect to the terms used in the present invention, general terms currently and widely used are selected in consideration of function in the present invention, but the terms may vary according to an intention of a technician skilled in the art, a precedent, the advent of a new technology, and the like. Further, in specific cases, there is also a term arbitrarily chosen by the applicant, and in this case, the meanings thereof will be described in detail in the corresponding part of the Detailed Description of the present invention. Accordingly, the term used in the present invention should not be defined merely as a simple name of the term, but should be defined based on the meaning of the term and overall content of the present invention.

Throughout the specification of the present invention, when one part "comprises" one constituent element, unless otherwise specifically described, this does not mean that another constituent element is excluded, but means that another constituent element may be further comprised. Throughout the specification of the present invention, a term of a degree, such as "about" or "substantially," is used as meaning a corresponding numerical value or used as a meaning close to the numerical value when a natural manufacturing and a material tolerance error are presented in a described meaning, and is used to prevent an unconscientious infringer from illegally using disclosed contents comprising a numerical value illustrated as being accurate or absolute in order to help understanding of the present invention.

Throughout the specification of the present invention, the term "combination thereof' comprised in the Markush type expression means a mixture or combination of one or more selected from the group consisting of constituent elements described in the Markush type expression, and means comprising one or more selected from the group consisting of the above-described constituent elements.

As used herein, "expression suppression" refers to all actions that reduce the expression of a gene, and may be achieved by gene knock-out, knock-down, or introducing mutations such as deletion, duplication, inversion, or translocation into gene DNA sequences, but is not limited thereto.

As used herein, "activity suppression" refers to the suppression of one or more functions caused by cathepsin S, and the function may be a function related to the occurrence/aggravation of symptoms of age-related macular degeneration such as causing age-related macular degeneration, progressing or exacerbating symptoms of age-related macular degeneration, or increasing the rate of progression of age-related macular degeneration, but is not limited thereto.

Hereinafter, preferred examples for helping with understanding of the present invention will be suggested. However, the following examples are provided only so that the present invention may be more easily understood, and the content of the present invention is not limited by the following examples.

### [Description of Drawings]

FIG. 1A is a graph showing a viability of RPE cells according to H₂O₂ treatment.
FIG. 1B and FIG. 1C are graphs showing iNOS expression levels in RPE cells according to H₂O₂ treatment and photographs showing Western blot results.
FIG. 2A and FIG. 2B are graph showing the expression level of cathepsin S in RPE cells according to H₂O₂ treatment and photograph showing Western blot results.
FIG. 3A and FIG. 3B are graph showing cathepsin S expression levels in RPE cells and cathepsin S knockdown RPE cells following H₂O₂ treatment, and photograph showing immunofluorescence staining results.
FIG. 4A to FIG. 4C are graphs showing inflammatory cytokine expression levels in RPE cells and cathepsin S knockdown RPE cells according to H₂O₂ treatment, and FIG. 4D is a result of analyzing each expression level by Western blotting.
FIG. 5A to FIG. 5B are graphs showing complement expression levels in RPE cells and cathepsin S knockdown RPE cells according to H₂O₂ treatment, and FIG. 5C and FIG. 5D show the results of analyzing each expression level by Western blotting.
FIG. 6A and FIG. 6B are immunofluorescence staining results showing the complement activity inhibitory effect upon treatment of RPE cells with CTSS siRNA.
FIG. 7Ais a photograph showing the results of Western blot for the expression level of angiogenic factors in RPE cells according to H₂O₂ treatment and cathepsin S knockdown RPE cells by CTSS siRNA.
FIG. 7B is an inverted fluorescence micrograph showing the tube formation reduction effect in RPE cells according to H₂O₂ treatment and cathepsin S knockdown RPE cells by CTSS siRNA treatment.
FIG. 8 is a photograph showing the effect of reducing choroidal neovascularization when CTSS siRNA is treated in a mouse model of choroidal neovascularization.

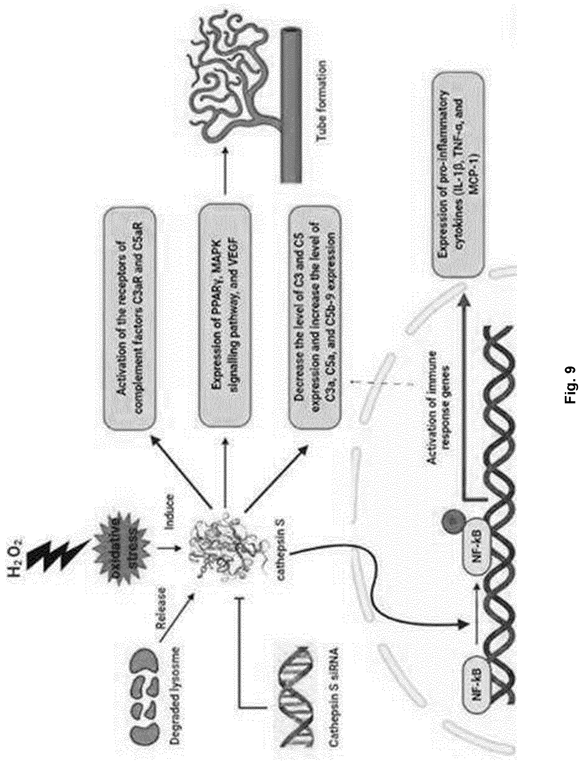

FIG. 9 is a schematic diagram showing the mechanism of cathepsin S in age-related macular degeneration.

### [Examples]

### Example 1. Confirmation of decrease in viability of RPE cells according to H₂O₂ treatment

In order to confirm the decrease in viability of retinal pigment epithelium cells (RPE cells, hereinafter referred to as RPE cells) according to H₂O₂ treatment, MTT analysis was performed, and the viability and iNOS expression of RPE cells according to H₂O₂ treatment were confirmed.

First, a human retinal-pigmented epithelial cell line (hereinafter referred to as ARPE-19 cells (ATCC, Mansas, VA, USA)) was used and inoculated into 96-well plates and cultured for 24 hours. Thereafter, the cells were treated with 6.25 µM 12.5 µM 25 µM, 50 µM, and 100 µM H₂O₂ for 48 hours, a 3-(4,5-dimethylthiazol-2-yl)-2,5-diphenyl-tetrazolium bromide (MTT) solution was added to the medium, and the cells were cultured at 37°C for 4 hours. The MTT-comprising medium was removed and the cells were lysed in 50 µl of DMSO. An optical density at 540 nm was confirmed using a microplate spectrophotometer (SpectraMax; Molecular Devices, Sunnyvale, CA, USA), and cell viability was determined by repeating each experiment three times to calculate an average value.

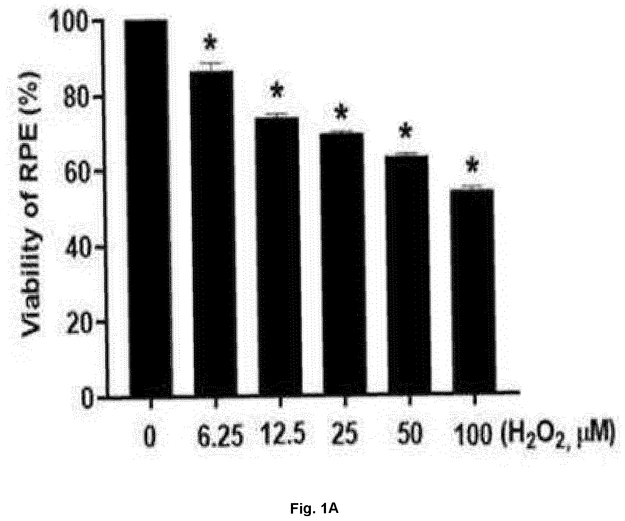

As a result, a decrease in cell viability was shown in a group treated with H₂O₂ compared to the control, confirming that H₂O₂ acts as a stress that can affect RPE cell survival (FIG. 1A).

In addition, the expression level of iNOS expressed in RPE cells according to H₂O₂ treatment was analyzed. Specifically, after RPE cells were treated with 10 µM, 50 µM, and 100 µM H₂O₂, relative iNOS mRNA expression levels were confirmed, and western blot experiments were performed. Total RNA was prepared using a Ribospin^{™} II kit from GeneAll Biotechnology (Seoul, South Korea) according to the manufacturer's recommendations. Total RNA (1 µg) was reverse transcribed into first-strand cDNA using an iScript^{™} cDNA Synthesis kit (Bio-Rad Laboratories, Inc., California, USA) according to the manufacturer's instructions, real-time reverse transcription-polymerase chain reaction (RT-PCR) was performed using a Thermal Cycler CFX96^{™} Real-Time PCR System (Bio-Rad Laboratories, USA), and each experiment was repeated three times to derive the results. RPE cells were washed with ice-cold PBS and then extracted with a protein lysis buffer (Intron Biotechnology, Seoul, South Korea). Protein samples of cell lysates were mixed with an equal volume of 2×SDS sample buffer, denatured by heating for 5 minutes and then separated on a 10% SDS-PAGE gel. After electrophoresis, proteins were transferred to a polyvinylidene difluoride membrane, and the membrane was blocked and washed using 5% BSA for 40 minutes. Thereafter, the membrane was incubated with specific antibodies against iNOS and β-actin in Tris-buffered saline (TBS) comprising Tween-20 (TBS-T, 0.1%) at 4°C overnight. In this case, β-actin was used as a loading control. A primary antibody was washed on the membrane using TBS-T (X3) and the membrane was incubated with a horseradish peroxidase-conjugated secondary antibody (1:1000-2000) for 2 hours. After washing three times with TBS-T, immunopositive bands were visualized using the ChemiDocTMXRS+ imaging system with Image Lab^{™} software (Bio-Rad Laboratories, USA) and the average of results from experiments each repeated three times was derived.

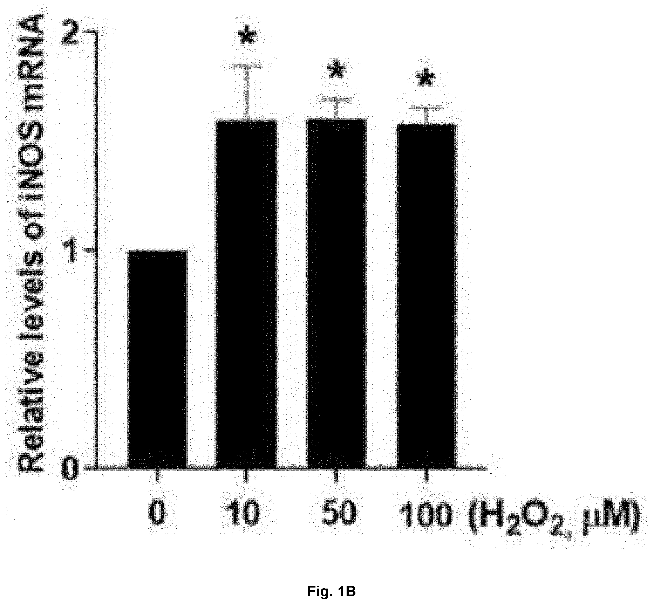

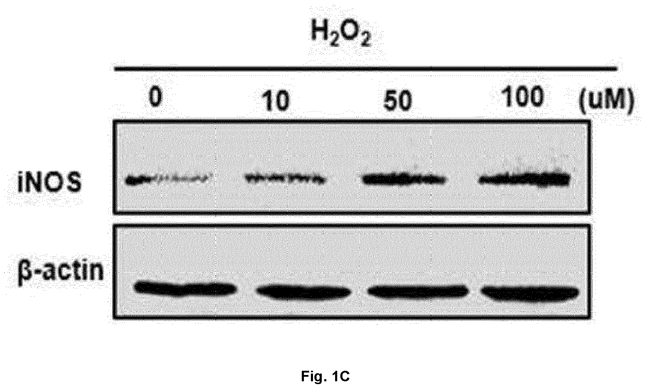

As a result, it was shown that the relative iNOS mRNA levels in RPE cells increased upon treatment with 50 µM and 100 µM H₂O₂ (FIG. 1B). In addition, as a result of a western blot experiment, it was confirmed that the iNOS protein increased in a H₂O₂ concentration-dependent manner (FIG. 1C). That is, it was confirmed that H₂O₂ acts as oxidative stress on RPE cells.

### Example 2. Confirmation of increase in expression of cathepsin S (CTSS) in RPE cells under oxidative stress conditions

To confirm the expression of cathepsin S (CTSS) in RPE cells under oxidative stress conditions, RPE cells were treated with H₂O₂ and the expression of cathepsin S (CTSS) was determined. Specifically, the cells was treated with 10 µM and 50 µM H₂O₂, cathepsin S mRNA expression level analysis and western blot experiments were performed in order to confirm the expression of cathepsin S, and the same experimental method and conditions as in Example 1 were applied, except that cathepsin S-specific antibodies were used.

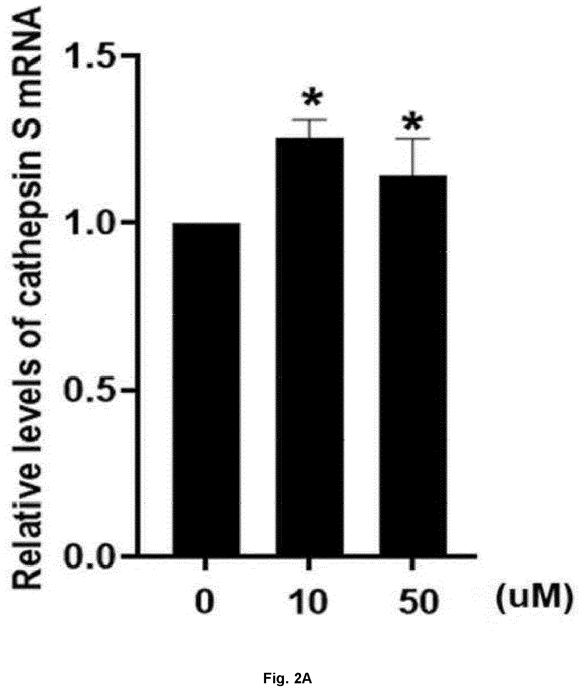

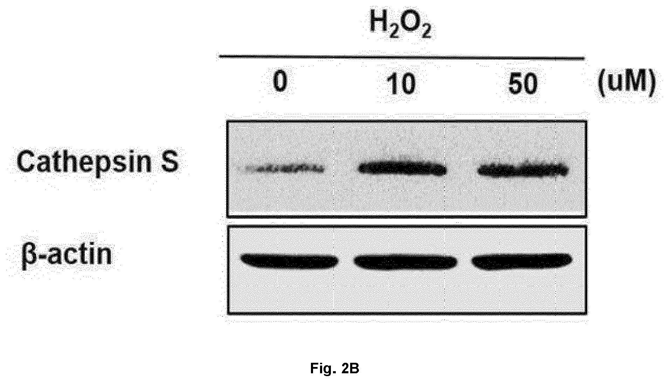

As a result, it was confirmed that the expression of cathepsin S increased when oxidative stress acted on RPE cells, in that upon treatment with H₂O₂, the relative mRNA level of cathepsin S in RPE cells was higher than that of the control (FIG. 2A) and western blot experimental results showed that the cathepsin S protein band also appeared thick and dark (FIG. 2B).

### Example 3. Confirmation of cathepsin S (CTSS) unresponsiveness in cathepsin S knockdown RPE cells under oxidative stress conditions

It was also analyzed whether cathepsin S expression was increased by oxidative stress in cathepsin S knockdown RPE cells.

### Example 3-1. Confirmation of CTSS mRNA expression level in cathepsin S knockdown RPE cells in response to H₂O₂

siRNA transfection was performed to prepare cathepsin S knockdown RPE cells. Specifically, cells were cultured to 50 to 60% confluence, and for a transfection mixture, Lipofectamine RNAiMAX (Invitrogen, Carlsbad, CA, USA), OPTI-MEM and CTSS siRNA (Santa Cruz sc-29940) were cultured. After culturing for 10 minutes, cells were treated according to manufacturer's instructions, cultured for 24 hours and transfected, cultured (70 to 80% confluence), then treated with 50 µM H₂O₂ as indicated and cultured for an additional 48 hours. Thereafter, RT-PCR was performed according to the protocol given and the average of results from experiments each repeated three times was derived. In this case, β-actin was used as a loading control, cathepsin S knockdown RPE cell lines were prepared by infecting ARPE-19 cells with control siRNA (control) or the CTSS siRNA for 24 hours, and the relative mRNA expression levels of cathepsin S were analyzed by treating the control and cathepsin S knockdown RPE cell lines with H₂O₂. The mRNA expression level was measured by applying the same conditions and method as in Example 2.

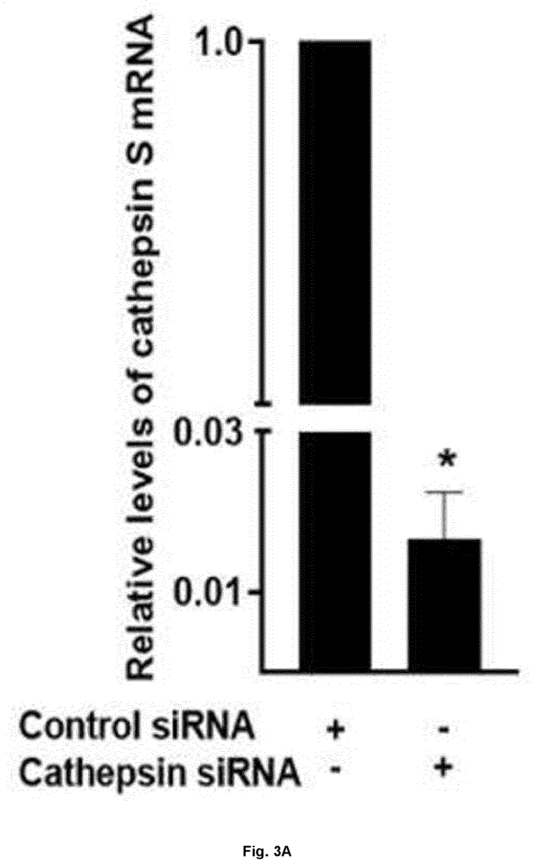

As a result, it was shown that the relative expression level of cathepsin S mRNA in cathepsin S knockdown RPE cells was about 1/100 of that of the control, confirming that the expression of cathepsin S in RPE cells was remarkably suppressed despite H₂O₂ treatment (FIG. 3A).

### Example 3-2. Immunofluorescence analysis of cathepsin S knockdown RPE cells in response to H₂O₂

Immunofluorescence analysis was performed to confirm the suppression of cathepsin S in cathepsin S knockdown RPE cells of Example 3-1. Specifically, after cells prepared in the same manner as in the mRNA expression level analysis were washed twice with sterile PBS and fixed with 4% paraformaldehyde at room temperature for 30 minutes, the fixation was stopped by washing the cells three times with PBS. The cells were permeabilized with PBS comprising 0.01% Triton X-100 for 10 minutes. After blocking for 1 hour, the insert membrane was removed using a scalpel and placed on Parafilm prior to being incubated with a primary antibody (depending on the protein of interest). All primary antibodies were diluted 1:50 in 5% BSA and PBS and used, and cultured at 4°C overnight. Thereafter, the cells were washed three times with PBS and a secondary FITC- or TRITC-conjugated antibody was diluted 1:50 and applied at room temperature for 1 hour. After 1 mg/ml DAPI was added 10 minutes before removing a secondary antibody to stain the nuclei, the cells were coated with a mounting medium and a glass coverslip was placed on top. The membrane/coverslip was fixed to the glass slide using a mounting medium, and cells were observed under a fluorescence microscope (Leica DFC7000T).

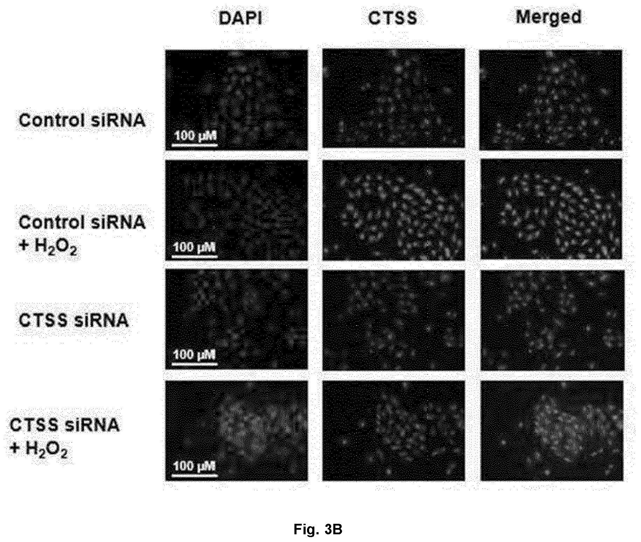

As a result, when a fluorescent probe was used, H₂O₂ treatment significantly increased cathepsin S expression, which was confirmed by bright red fluorescence. (FIG. 3B). As a result of analyzing RPE cells exposed to H₂O₂ (50 µM, 48 hours) or a control after transfection with the control siRNA or CTSS siRNA for 24 hours, cathepsin S was significantly decreased in CTSS siRNA + H₂O₂ compared to the control siRNA + H₂O₂ (p<0.05).

According to these results, it was confirmed that when cathepsin S was knocked down, despite the H 2 O 2 treatment, the expression of cathepsin S induced by this was greatly reduced and the red fluorescence signal was reduced.

### Example 4. Confirmation of inflammatory cytokine reduction in cathepsin S knockdown RPE cells

To confirm the inflammatory cytokine reduction effect was confirmed in cathepsin S knockdown RPE cells. Specifically, the RPE cells were treated with H₂O₂, and then the expression of p-NF-κB (the active form of NF-κB) and inflammatory cytokines was analyzed by RT-PCR. The same experimental group cells (cathepsin S knockdown RPE cells) and control cells as in Example 3 were used, and each cell was treated with H₂O₂ under the same conditions as in Example 3 (50 µM H₂O₂, 48 hours). Thereafter, the RT-PCR and western blot experimental methods and conditions for confirming the expression of p-NF-κB and inflammatory cytokines were applied in the same manner as in Example 1 or 2. In this case, NF- κB and β-actin were used as loading controls.

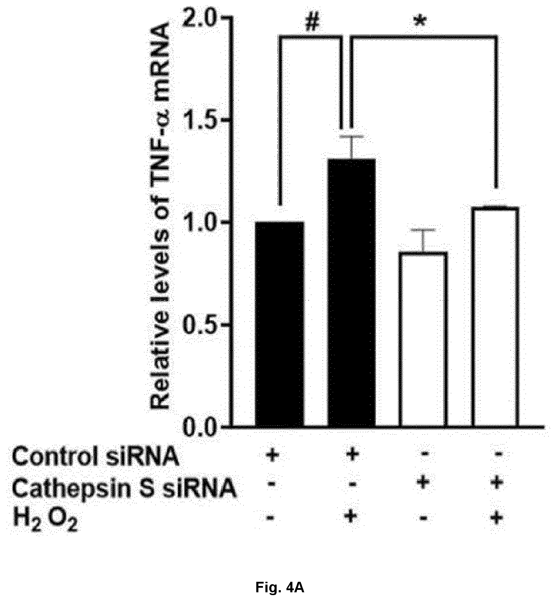

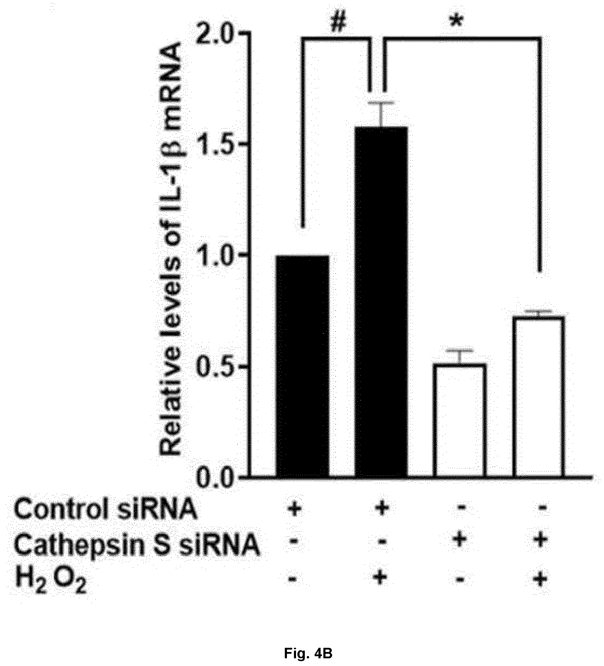

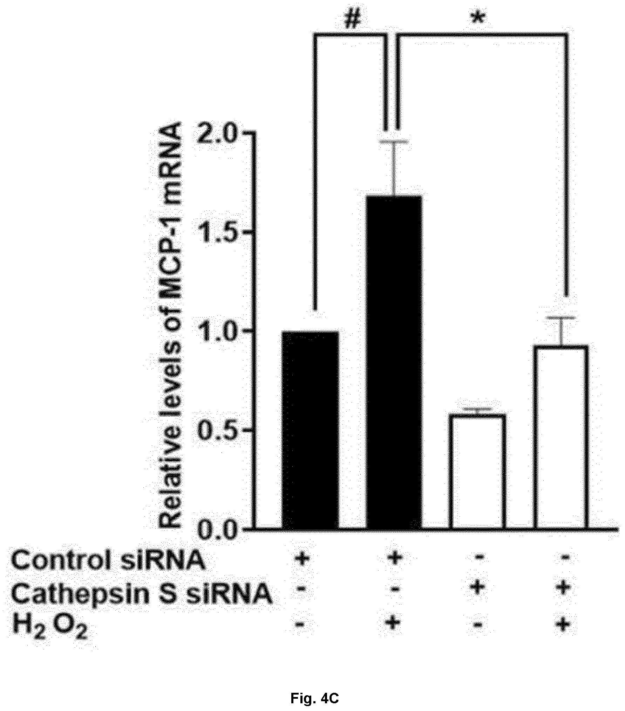

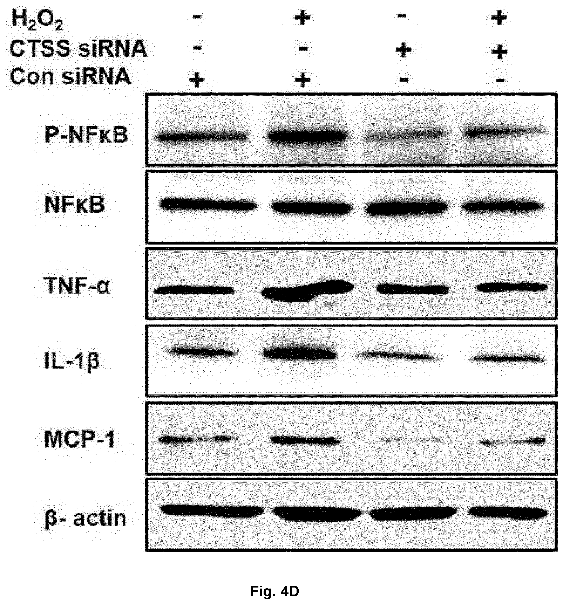

As a result, the relative TNF-α, IL-1β, and MCP-1 expression levels in the control were remarkably higher than those in the experimental group upon H₂O₂ treatment (FIGS. 4A to FIGS. 4C). In addition, the expression of p-NF- κB, TNF-α, IL-1β, and MCP-1 proteins induced by H₂O₂ was reduced by cathepsin S knockdown (FIG. 4D). According to these results, it was analyzed that cathepsin S suppresses the expression of inflammatory cytokines TNF-α, IL-1β and MCP-1 in RPE cells at the transcriptional level.

### Example 5. Confirmation of complement factor expression reduction in cathepsin S knockdown RPE cells

The effect of reducing complement factor expression in cathepsin S knockdown RPE cells was confirmed. Specifically, after treating RPE cells with H₂O₂, the complement factor expression level was examined. The expression of C3aR and C5aR, which are receptors for C3a and C5a among complement factors, was analyzed by RT-PCR, and the protein expression of C3 (C3a), C5 (C5a), C3aR, C5aR and the active form of membrane attack complex C5b-9 was analyzed by Western blotting. It was analyzed through experiments. At this time, the same experimental group cells (cathepsin S knockdown RPE cells) and control cells as in Example 3 were used, and each cell was treated with H₂O₂ under the same conditions as in Example 3 (50 µM, 48 h). In addition, the experimental methods and conditions for RT-PCR and Western blot were applied in the same manner as in Example 1 or 2.

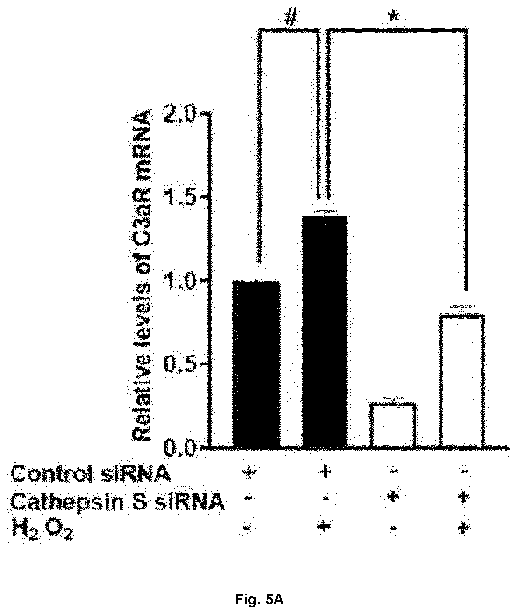

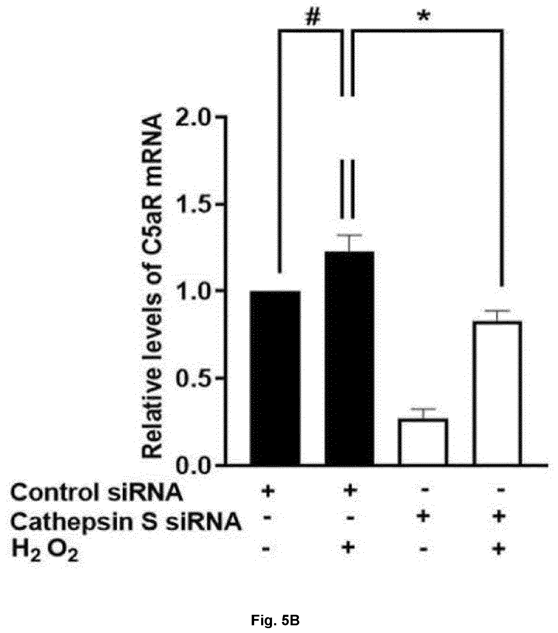

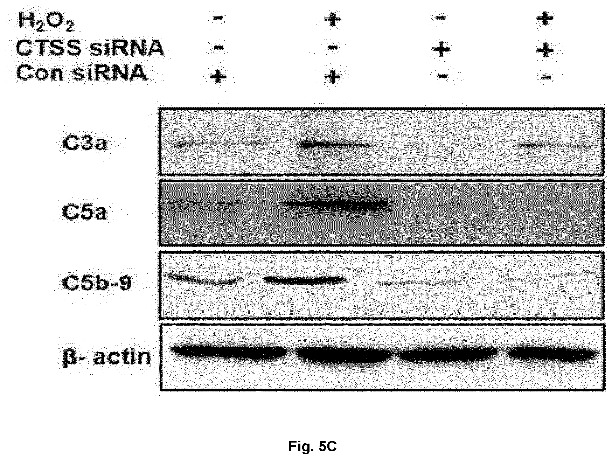

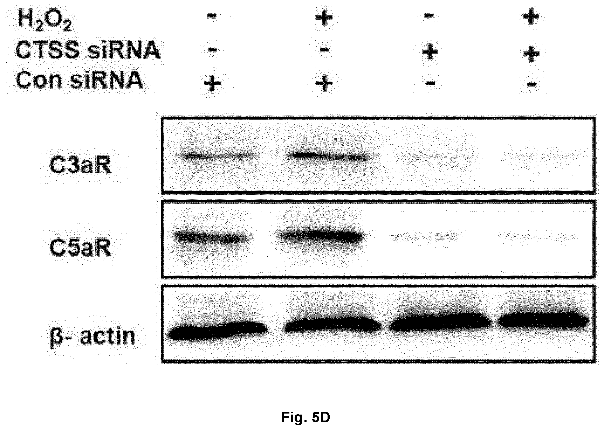

As a result, the relative C3aR and C5aR expression levels in the control were remarkably higher than those in the experimental group upon H₂O₂ treatment (FIGS. 5Ato 5B). Furthermore, it was shown that the expression of C3 (C3a), C5 (C5a), C3aR, C5aR and C5b-9 proteins induced by H₂O₂ was reduced by cathepsin S knockdown (FIGS. 5C and 5D), confirming that cathepsin S knockdown has a statistically significant effect of suppressing the complement (p<0.05). According to these results, it was analyzed that cathepsin S suppresses the complement system in RPE cells.

According to Examples 1 to 5, as the viability of RPE cells decreased and the expression of cathepsin S increased during H₂O₂ treatment, the correlation between H₂O₂ and cathepsin S expression was confirmed. In addition, it was confirmed that when cathepsin S in RPE cells was knocked down, the expression of cathepsin S was suppressed despite H₂O₂ treatment, and inflammatory cytokines and complement activity were suppressed, thereby reducing the inflammatory response. Accordingly, it was confirmed that inhibition of cathepsin S is important in age-related macular degeneration, and it was analyzed whether the same result was obtained by treating cathepsin S expression or an inhibitor.

### Example 6. Confirmation of excellent complement activity suppression effect of cathepsin S siRNA

In order to confirm the inhibitory effect of complement activity in the case of treatment with an inhibitor of cathepsin S expression or activity, RPE cells were treated with cathepsin S siRNA (CTSS siRNA, CTSSiRNA) and then the effect of inhibiting complement activity was analyzed. At this time, NF-κB inhibitor were treated as a comparison group. Specifically, RPE cells were treated with control siRNA or CTSS siRNA for 24 hours in the presence or absence of NF-κB inhibitor Bay 1170-82 (10 µM), respectively. After that, immunofluorescence analysis was performed for the expression of C3a and C5a in RPE cells. Experimental methods and conditions were applied in the same manner as in Example 3.

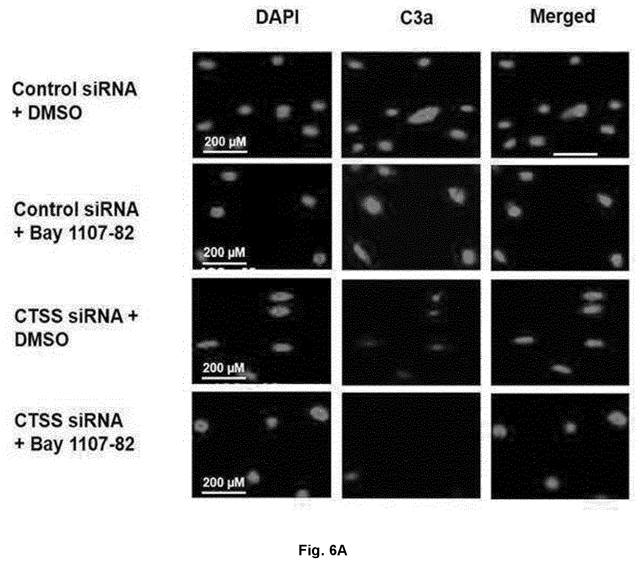

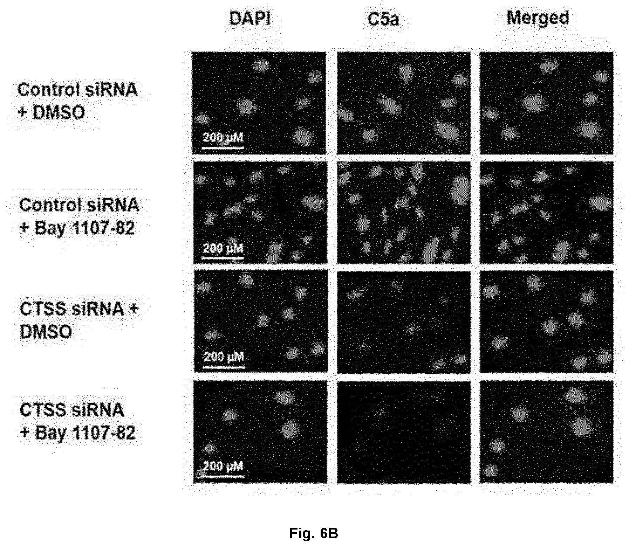

As a result, upon treatment with Bay 1170-82, it was shown that red fluorescence signals decreased and the expression of C3a and C5a was suppressed compared to the control DMSO, confirming that the C3a and C5a expression suppression effects of CTSS siRNA are remarkably excellent compared to those of Bay 1170-82 (FIGS. 6A and FIGS.6B). Further, it was shown that simultaneous treatment with CTSS siRNA and an NF-κB inhibitor decreased C3a and C5a expression more than CTSS siRNA or the NF-κB inhibitor alone, confirming that CTSS siRNA has a synergistic effect in NF-κB inhibitor and complement activity suppression. According to these results, it is analyzed that cathepsin S is able to act in the same pathway as NF-κB and act as a regulator of complement activation.

### Example 7. Confirmation of excellent reduction in angiogenesis in cathepsin S siRNA

In the case of treatment with an inhibitor of cathepsin S expression or activity, it was confirmed whether angiogenesis in RPE cells induced by oxidative stress was reduced. Specifically, the expression levels of PPARγ and VEGFA/AKT, which play key roles in neovascularization, differentiation, migration, and proliferation, were confirmed through western blotting experiments, and the experimental methods and conditions were applied in the same manner as in Example 2. In addition, to confirm the presence or absence of cell tube formation, HUVECs (1 × 10⁵) were treated on a Matrigel-coated 96-well-plate with reduced growth factors, and then the cells were treated with a conditioned medium (CM) for 2 hours by treating RPE cells with CTSS siRNA and H₂O₂ at 37°C for 48 hours, and images were analyzed with an inverted fluorescence microscope.

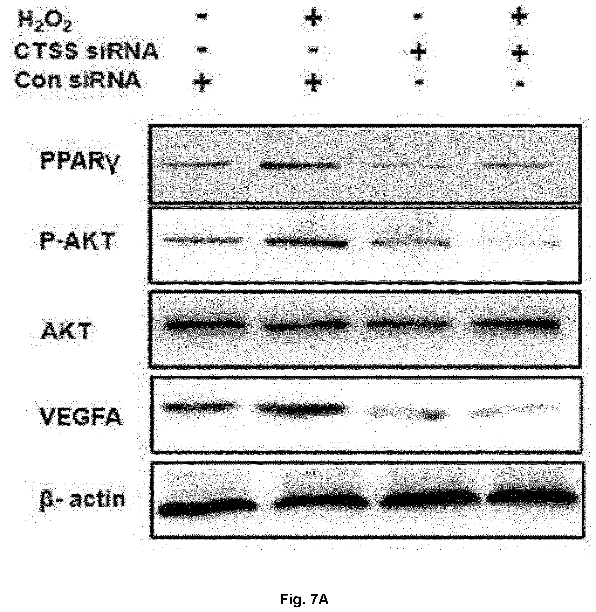

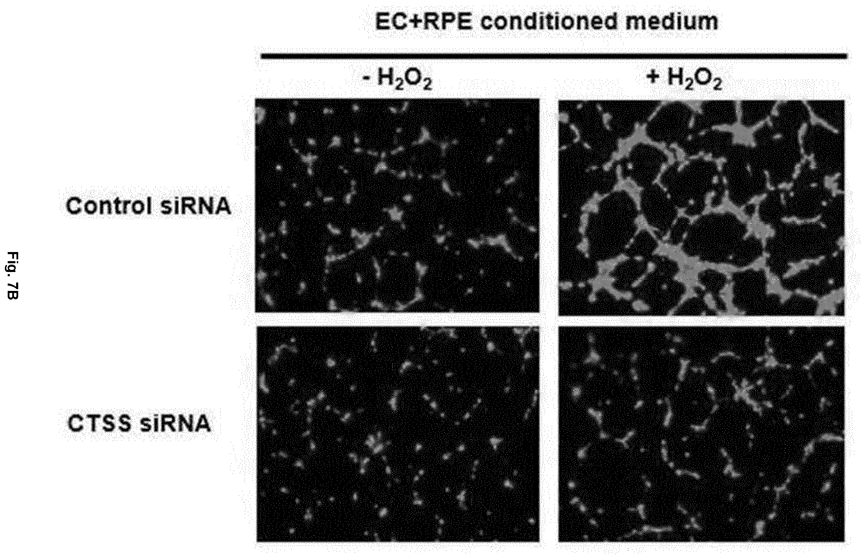

As a result, the expression levels of PPARγ and VEGFA/AKT (p-AKT) increased when treated with H₂O₂, whereas when treated with CTSS siRNA, the expression levels of PPARγ and VEGFA/AKT (p-AKT) compared to the control group despite oxidative stress conditions were found to decrease significantly (Fig. 7A). In addition, unlike the marked increase in tube formation in the control group when treated with H₂O₂, it was confirmed that tube formation was significantly inhibited in the case of CTSS siRNA treatment than in the untreated control group (FIG. 7B).According to these results, CTSS siRNA was found to have excellent preventive and therapeutic effects on age-related macular degeneration, which is the main cause of neovascular hyperplasia, in that not only neovascularization but also intracellular tube formation were significantly inhibited in RPE cells treated with CTSS siRNA..

### Example 8. Confirmation of excellent angiogenesis reduction effect of cathepsin S siRNA in choroidal neovascularization (CNV) model

The effect of cathepsin S siRNA on choroidal neovascularization in vivo was confirmed. First, in order to confirm the in vivo effect, a choroidal neovascularization (CNV) model was first constructed. The animal research protocol was approved by the Animal Care and Use Committee of Chung-Ang University (approval number, A2021042). All animal experiments were conducted in accordance with the guidelines of the ARVO Statement for the Use of Animals in Ophthalmic and Vision Science Research. Choroidal neovascularization in the mouse model was induced by laser. Six-week-old male C57BL/6J mice (Orient Co., Sungnam, Korea) were anesthetized with intraperitoneal injection of 240 mg/kg avertin (2-2-2-tribromoethanol 1.2%) (Papioannu and Fox, 1993). Diode laser at 12 and 6 o'clock positions, 2 to 3 disc diameter (75 µm spot size, 120 mW, 100 ms) on an optical disc, using a slit lamp delivery system (OcuLight Tx, IRIDEX, CA, USA) with a round coverslip. Three retinal burn lesions were induced in the right eye of each mouse (n=10). Formation of bubble was considered a valid burn and a sign of Bruch's membrane rupture. After 36 hours, siRNA diluted in RNAse-free water was injected into both eyes using a 5uL syringe (Hamilton Co, Reno, NV, NV, USA) and a 32-gauge needle at a distance of 0.5 mm from the limbus ( 5 µL [15 nmol]/eye). Mouse Cts (mCts) siRNA (n=5) and control siRNA (n=5) (both Santa Cruz Biotechnology) were injected into the right eye, respectively. After 7 days of laser application, the eyeball was removed and fixed with 4% paraformaldehyde. An anterior segment was removed to obtain a retina/choroid area. The choroid/retinal pigment epithelium complex was immunohistochemically stained as follows. The choroid/retinal pigment epithelium complex was reacted overnight with an anti-CD31 antibody at a dilution of 1:100, and choroidal neovascularization was visualized under a fluorescence microscope using secondary Alexa Fluor 594 conjugated goat anti-mouse IgG (provided by Invitrogen).

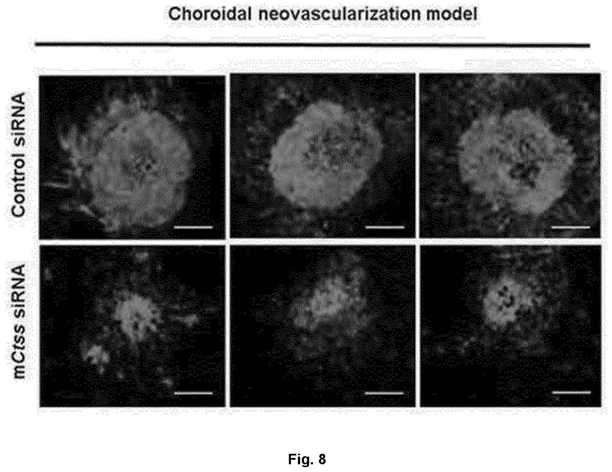

As a result, in the choroidal neovascularization mouse model, it was confirmed that the amount of choroidal neovascularization after injection of CTSS siRNA was significantly more reduced than after injection of control siRNA (FIG. 8).

Overall, it was confirmed that cathepsin S was induced in RPE cells by H₂O₂ (Example 2) and inflammatory cytokines (Examples 3 and 4) and complement activity (Example 5) were remarkably suppressed in a cathepsin S knockdown RPE model, a complement activation suppression effect by CTSS siRNA is not only excellent compared to that of an NF-κB inhibitor (Example 6), but also remarkably reduces neovascularization and tube formation (Example 7). These experimental results are also shown in the choroidal neovascularization mouse model (Example 8), and a substance that reduces the expression or activity of cathepsin S, for example, CTSS siRNA, is expected to be utilized as a preventive or therapeutic target for age-related macular degeneration that may be used for both dry and wet age-related macular degeneration.

The above-described description of the present invention is provided for illustrative purposes, and those skilled in the art to which the present invention pertains will understand that the present invention can be easily modified into other specific forms without changing the technical spirit or essential features of the present invention. Therefore, it should be understood that the above-described Examples are illustrative only in all aspects and are not restrictive.

## Claims

1. A composition comprising a material that reduces the expression or activity of cathepsin S (CTSS) as an active ingredient for use in the treatment of age-related macular degeneration.

2. The composition of claim 1, wherein the substance is any one or more selected from the group consisting of small interfering RNA (siRNA), short hairpin RNA (shRNA), micro RNA (miRNA), antisense oligonucleotides (ASOs), Clustered Regularly Interspaced Short Palindromic Repeats (CRISPR/Cas), natural products, proteins, peptidomimetics, antibodies, exosomes, and compounds.

3. The composition of claim 1, wherein the age-related macular degeneration may be age-related macular degeneration under oxidative stress conditions.

4. The composition of claim 1, wherein the age-related macular degeneration may be dry age-related macular degeneration or wet age-related macular degeneration.

5. The composition of claim 1, wherein the composition may be **characterized by** any one or more of the following:
inhibition of inflammatory cytokines and complement activity in the retina;
inhibition of intravascular endothelial production; and
inhibition of angiogenesis.

6. The composition of claim 5, wherein the inflammatory cytokine is any one or more selected from the group consisting of TNF-α, IL-1β, and MCP-1.

7. The composition of claim 5, wherein the complement is any one or more selected from the group consisting of C3, C5, C3a, C5a, and C5b-9.

8. An ophthalmic composition for preventing or treating age-related macular degeneration, comprising, as an active ingredient, a substance that reduces the expression or activity of cathepsin S (CTSS).

9. The composition of claim 8, wherein the composition may be an eye drop composition.

10. A health functional food composition for preventing or alleviating age-related macular degeneration, comprising, as an active ingredient, a substance that reduces the expression or activity of cathepsin S (CTSS).

11. The composition of claim 10, wherein the material is any one or more selected from the group consisting of small interfering RNA (siRNA), short hairpin RNA (shRNA), micro RNA (miRNA), antisense oligonucleotides (ASOs), Clustered Regularly Interspaced Short Palindromic Repeats (CRISPR/Cas), natural products, proteins, peptidomimetics, antibodies, exosomes, and compounds.

12. A kit for preventing or treating age-related macular degeneration, comprising a composition comprising, as an active ingredient, a substance that reduces the expression or activity of cathepsin S, and instructions.

13. A use of a material that reduces the expression or activity of cathepsin S (CTSS) for the manufacture of a medicament for treating age-related macular degeneration.
